# EUROPEAN PATENT APPLICATION

(11) **EP 3 939 587 A1**
(43) Date of publication of application: **19.01.2022**
(21) Application number: 21195602.4
(22) Date of filing: 22.05.2018
(51) Int. Cl.: A61K 31/437, A61K 9/08, A61K 31/424, A61K 31/435, A61K 31/4353, A61K 31/4355, A61K 47/02, A61K 9/00, A61P 25/24

(54) **GABOXADOL FOR USE IN THE TREATMENT OF DEPRESSION ASSOCIATED WITH FMR1-PREMUTATION SYNDROME**

(30) Priority: 24.05.2017 US 201762510481 P; 23.03.2018 US 201815933673
(62) Divisional of application: 18805653.5
(71) Applicant: Ovid Therapeutics, Inc., New York, NY 10036 (US)
(72) Inventor: During, Matthew, Weston, 06883 (US)
(74) Representative: Frick, Robert

(57) **Abstract**

The invention relates to a composition comprising gaboxadol or a pharmaceutically acceptable salt thereof for use in a method of treating FMR1-premutation syndrome, the method comprising administering the composition to a patient in need thereof such as to administer to the patient 1 mg to 30 mg gaboxadol or pharmaceutically acceptable salt.

## Description

### CROSS REFERENCE TO RELATED APPLICATIONS

This application claims benefit of and priority to U.S. Provisional Application No. 62/510,481, filed May 24, 2017 and U.S. Patent Application No. 15/933,673, filed March 23, 2018, which are incorporated herein by reference in their entirety.

### TECHNICAL FIELD

Formulations of gaboxadol or pharmaceutically acceptable salts thereof useful for depressive disorders are provided.

### BACKGROUND

Parenteral dosage forms are intended for administration as an injection or infusion. Common injection types are intravenous (into a vein), subcutaneous (under the skin), and intramuscular (into muscle). Infusions typically are given by intravenous route. Parenteral formulations often include excipients to enhance or maintain active ingredient solubility (solubilizers) and/or stability (buffers, antioxidants, chelating agents, cryo- and lyoprotectants). Excipients also are important in parenteral formulations to assure safety (antimicrobial preservatives), minimize pain and irritation upon injection (tonicity agents), and control or prolong drug delivery (polymers). However, excipients may also produce negative effects such as loss of drug solubility, activity, and/or stability.

Gaboxadol (4,5,6,7-tetrahydroisoxazolo [5,4-c] pyridine-3-ol) (THIP)) is described in U.S. Patent Nos. 4,278,676, 4,362,731, 4,353,910, and WO 2005/094820 is a selective GABA_{A} receptor agonist with a preference for δ-subunit containing GABA_{A} receptors. In the early 1980s gaboxadol was the subject of a series of pilot studies that tested its efficacy as an analgesic and anxiolytic, as well as a treatment for tardive dyskinesia, Huntington's disease, Alzheimer's disease, and spasticity. In the 1990s gaboxadol moved into late stage development for the treatment of insomnia but failed to show significant effects in sleep onset and sleep maintenance in a three-month efficacy study. Additionally, patients with a history of drug abuse who received gaboxadol experienced a steep increase in psychiatric adverse events. As a result of these negative results the development of gaboxadol was terminated.

Depressive disorders are one of the most prevalent and pervasive forms of mental illness that affect individuals across age and gender lines. There remains a need in the art for safe and effective pharmaceutical compositions that may provide treatment of depressive disorders. Depressive disorders are mood disorders characterized by sadness or lethargy severe enough to interfere with normal daily functioning. Symptoms include depressed mood for most or all of the day, anxious or sad feelings, diminished interest in all or nearly all activities, significant increased or decreased appetite leading to weight gain or weight loss, insomnia, irritability, fatigue, feelings of worthlessness, feelings of helplessness, inability to concentrate, and recurrent thoughts of death or suicide to name a few. The presence, severity, frequency, and duration of the above mentioned symptoms vary on a case to case basis. To be diagnosed with depression, symptoms are typically present for at least two weeks. Depressive disorders include major depressive disorder (MDD), persistent depressive disorder, postpartum depression, premenstrual dysphoric disorder, seasonal affective disorder, psychotic depression, disruptive mood dysregulation disorder, refractory depression and depressive disorders due to another medical condition.

Major depressive disorder (MDD), also referred to as unipolar or major depression, is a depressive disorder characterized by a persistent feeling of sadness or a lack of interest in outside stimuli. In MDD symptoms of depression, such as those described above, may be manifest daily or almost daily, and may persist all day long. Persistent depressive disorder (also called dysthymia) is a depressed mood that can last for at least about two years. A person diagnosed with persistent depressive disorder may have episodes of major depression along with periods of less severe symptoms. Postpartum depression occurs in pregnancy after delivery. Women with postpartum depression experience full-blown major depression. The feelings of extreme sadness, anxiety, and exhaustion that accompany postpartum depression may make it difficult for these new mothers to complete daily care activities for themselves and/or for their babies. Premenstrual dysphoric disorder involves mood and anxiety symptoms that are related to the menstrual cycle, with onset during the premenstrual phase and a symptom-free interval after menstruation. Symptoms are present during most menstrual cycles during the past year. Manifestations can be similar to those of premenstrual syndrome but are more severe, causing clinically significant distress and/or marked impairment of social or occupational functioning. The disorder may begin any time after menarche; it may worsen as menopause approaches but usually ceases after menopause. Seasonal affective disorder (SAD) is categorized as depression directly caused by the time of the year. It occurs most often in the fall or winter months when sunlight is not as readily available. Psychotic depression is depression accompanied by delusions or hallucinations. Delusions may involve having committed unpardonable sins or crimes, harboring incurable or shameful disorders, or being persecuted. Disruptive mood dysregulation disorder (DMDD) is a childhood condition of extreme irritability, anger, and frequent, intense temper outbursts. DMDD symptoms typically go beyond a being a "moody" child, i.e., children with DMDD experience severe impairment that requires clinical attention. Depressive disorders due to another medical condition may include those associated with chronic pain, chronic stress, post-traumatic stress disorder, and/or diseases, e.g., cancer, Parkinson's disease, amyotrophic lateral sclerosis, and multiple sclerosis.

Other medical conditions include *FMR1* premutation syndrome. It is caused by mutations on the fragile X mental retardation gene (*FMR1*) and lack of fragile X mental retardation protein, which in turn, leads to decreased inhibition of translation of many synaptic proteins. Fragile X syndrome (FXS) may be the most common genetic cause of intellectual disability and the most common single-gene cause of autism. Fragile X-associated tremor/ataxia syndrome (FXTAS) is a late-onset disorder, usually occurring after age 50. Mutations in the *FMR1* gene increase the risk of developing FXTAS. Similarly, mutations in the *FMR1* gene have been associated with Fragile X-associated primary ovarian insufficiency (FXPOI), a condition in which the ovaries are not functioning at full capacity. Both FXTAS and FXPOI are the result of *FMR1* premutation syndrome. The *FMR1* mutation relates to a DNA segment known as a CGG triplet repeat which is expanded within the *FMR1* gene. Normally, this DNA segment is repeated from 5 to about 44 times, with 45-54 repeats considered an intermediate zone. In people with *FMR1* premutation syndrome the CGG segment may be repeated 55 to 200 times. An expansion of more than 200 repeats, a full mutation, causes Fragile X syndrome mentioned above. Several additional medical, emotional and cognitive challenges, including depression, have also been described as occurring at a greater frequency among individuals with a premutation than would be expected in the general population. See, e.g., Wheeler et al., Journal of Neurodevelopmental Disorders 2014, 6:30.

Refractory depression occurs in patients suffering from depression who are resistant to standard pharmacological treatments, including tricyclic antidepressants, MAOIs, SSRIs, SNRIs, and double and triple uptake inhibitors and/or anxiolytic drugs, as well non-pharmacological treatments such as psychotherapy, electroconvulsive therapy, vagus nerve stimulation and/or transcranial magnetic stimulation. A treatment resistant-patient may be identified as one who fails to experience alleviation of one or more symptoms of depression (e.g., persistent anxious or sad feelings, feelings of helplessness, hopelessness, pessimism) despite undergoing one or more standard pharmacological or non-pharmacological treatment.

Accordingly, this disclosure provides pharmaceutical compositions and methods that may be used in treating depressive disorders such as major depressive disorder, persistent depressive disorder, postpartum depression, premenstrual dysphoric disorder, seasonal affective disorder, psychotic depression, disruptive mood dysregulation disorder, refractory depression and depressive disorders due to another medical condition.

### SUMMARY

Provided herein are formulations of gaboxadol or pharmaceutically acceptable salts thereof, including parenteral formulations, for use in treating depressive disorders. Also provided are methods of treating depressive disorders, including major depressive disorder, persistent depressive disorder, postpartum depression, premenstrual dysphoric disorder, seasonal affective disorder, psychotic depression, disruptive mood dysregulation disorder and/or depressive disorders due to another medical condition, with formulations of gaboxadol. Also provided herein are methods of treating depression in treatment resistant patients or treating refractory depression.

### BRIEF DESCRIPTION OF THE DRAWING

Figure 1 shows both the theoretical and measured solubility of gaboxadol at different pH values.

### DETAILED DESCRIPTION

Pharmaceutical compositions of gaboxadol and pharmaceutically acceptable salts thereof are provided herein for use in treating depressive disorders including major depressive disorder, persistent depressive disorder, postpartum depression, premenstrual dysphoric disorder, seasonal affective disorder, psychotic depression, disruptive mood dysregulation disorder and/or depressive disorders due to another medical condition. Also provided herein are methods of treating refractory depression, e.g., patients suffering from a depression disorder that does not, and/or has not, responded to adequate courses of at least one, or at least two, other antidepressant compounds or therapeutics. As used herein "depressive disorder" encompasses refractory depression.

In embodiments, methods are provided for treating depressive disorders, e.g., major depressive disorder, persistent depressive disorder, postpartum depression, premenstrual dysphoric disorder, seasonal affective disorder, psychotic depression, disruptive mood dysregulation disorder, and/or depressive disorders due to another medical condition, by administering to the patient a pharmaceutical composition of gaboxadol or a pharmaceutically acceptable salt thereof. In embodiments, methods are provided for treating refractory depression by administering to the patient a pharmaceutical composition of gaboxadol or a pharmaceutically acceptable salt thereof. In embodiments, provided herein are methods of use for treating depressive disorders by administering a parenteral composition of gaboxadol or a pharmaceutically acceptable salt thereof to a patient in need thereof. In embodiments, provided herein are methods of use for treating refractory depression by administering a parenteral composition of gaboxadol or a pharmaceutically acceptable salt thereof to a patient in need thereof.

Described herein are methods of treating depressive disorders including major depressive disorder, persistent depressive disorder, postpartum depression, premenstrual dysphoric disorder, seasonal affective disorder, psychotic depression, disruptive mood dysregulation disorder, depressive disorders due to another medical condition, and/or refractory depression by administering to a patient in need thereof a pharmaceutical composition including gaboxadol or pharmaceutically acceptable salt thereof. Depressive disorders due to another medical condition include those associated with chronic pain, chronic stress, post-traumatic stress disorder, *FMR1* premutation syndrome and/or diseases, e.g., cancer, Parkinson's disease, amyotrophic lateral sclerosis, and multiple sclerosis.

In embodiments, a method of treating major depressive disorder includes administering to a patient in need thereof a pharmaceutical composition of gaboxadol or pharmaceutically acceptable salt thereof. In embodiments, a method of treating persistent depressive disorder includes administering to a patient in need thereof a pharmaceutical composition of gaboxadol or pharmaceutically acceptable salt thereof. In embodiments, a method of treating postpartum depression includes administering to a patient in need thereof a pharmaceutical composition of gaboxadol or pharmaceutically acceptable salt thereof. In embodiments, a method of treating premenstrual dysphoric disorder includes administering to a patient in need thereof a pharmaceutical composition of gaboxadol or pharmaceutically acceptable salt thereof. In embodiments, a method of treating seasonal affective disorder includes administering to a patient in need thereof a pharmaceutical composition of gaboxadol or pharmaceutically acceptable salt thereof. In embodiments, a method of treating psychotic depression includes administering to a patient in need thereof a pharmaceutical composition of gaboxadol or pharmaceutically acceptable salt thereof. In embodiments, a method of treating disruptive mood dysregulation disorder includes administering to a patient in need thereof a pharmaceutical composition of gaboxadol or pharmaceutically acceptable salt thereof. In embodiments, a method of treating depressive disorders due to another medical condition includes administering to a patient in need thereof a pharmaceutical composition of gaboxadol or pharmaceutically acceptable salt thereof. In embodiments, a method of treating depressive disorders due *FMR1* premutation syndrome includes administering to a patient in need thereof a pharmaceutical composition of gaboxadol or pharmaceutically acceptable salt thereof. In embodiments, a method of treating refractory depression includes administering to a patient in need thereof a pharmaceutical composition of gaboxadol or pharmaceutically acceptable salt thereof.

Many pharmaceutical products are administered as a fixed dose, at regular intervals, to achieve therapeutic efficacy. Its duration of action is reflected by its plasma half-life. Since efficacy is often dependent on sufficient exposure within the central nervous system administration of CNS drugs with a short half-life may require frequent maintenance dosing. Advantageously disclosed herein are methods of treating depressive disorders by administration of gaboxadol or pharmaceutically acceptable salt thereof. For example, in embodiments, methods of treating a depressive disorder are provided which include administering to a patient in need thereof a pharmaceutical composition including about 0.05 mg to about 75 mg of gaboxadol or pharmaceutically acceptable salt thereof, wherein the composition provides improvement for more than 6 hours after administration to the patient.

For example, dosages may include amounts of an gaboxadol or pharmaceutically acceptable salt thereof in the range of about, *e.g.,* 1 mg to 30 mg, 1 mg to 20 mg, 1 mg to 15 mg, 0.01 mg to 10 mg, 0.1 mg to 15 mg, 0.15 mg to 12.5 mg, or 0.2 mg to 10 mg, with doses of 0.1 mg, 0.2 mg, 0.3 mg, 0.4 mg, 0.5 mg, 0.6 mg, 0.7 mg, 0.8 mg, 0.9 mg, 1.5 mg, 1.0 mg, 1.75 mg, 2 mg, 2.5 mg, 2.75 mg, 3 mg, 3.5 mg, 3.75 mg, 4 mg, 4.5 mg, 4.75 mg, 5 mg, 5.5 mg, 6 mg, 6.5 mg, 7 mg, 7.5 mg, 8 mg, 8.5 mg, 9 mg, 10 mg, 11 mg, 12 mg, 15 mg, 20 mg, 25 mg, and 30 mg being specific examples of doses.

Typically, dosages of gaboxadol or a pharmaceutically acceptable salt thereof are administered once or twice daily to a patient in need thereof. The methods and compositions described herein may provide reduced dosing frequency and reduced adverse events and/or increased efficacy. In embodiments, the dosage is about, *e.g.,* 0.1-20 mg/day, or 0.2-15 mg/day, or 0.5-10 mg/day, or 0.75-5 mg/day, for example 0.2 mg/day, 0.5 mg/day, 0.75 mg/day, 1 mg/day, 1.5 mg/day, 2 mg/day, 3 mg/day, 4 mg/day, 5 mg/day, 6 mg/day, 7 mg/day, 8 mg/day, 9 mg/day, 10 mg/day, 11 mg/day, 12 mg/day, 13 mg/day, 14 mg/day, 15 mg/day, 16 mg/day, 17 mg/day, 18 mg/day, 19 mg/day, or 20 mg/day. In embodiments, gaboxadol or a pharmaceutically acceptable salt thereof, or a derivative or analogue thereof is administered at doses of 0.2 mg to 1 mg in infants or 1-20 mg in adults, once daily.

Methods of treating depressive disorders by administering to a subject in need thereof an effective amount of gaboxadol, a pharmaceutically acceptable salt thereof, derivative or analogue, or combination thereof, are provided. An effective amount or therapeutically effective amount can be a dosage sufficient to treat, inhibit, or alleviate one or more symptoms of a depressive disorder such as reducing the frequency or severity of sadness or lethargy, depressed mood, anxious or sad feelings, diminished interest in all or nearly all activities, significant increased or decreased appetite leading to weight gain or weight loss, insomnia, irritability, fatigue, feelings of worthlessness, feelings of helplessness, inability to concentrate, and recurrent thoughts of death or suicide, or to provide a desired pharmacologic and/or physiologic effect, for example, reducing, inhibiting, or reversing one or more of the underlying pathophysiological mechanisms underlying the neurological dysfunction, increasing dopamine levels or signaling, or a combination thereof. The precise dosage will vary according to a variety of factors such as subject-dependent variables (e.g., age, immune system health, clinical symptoms etc.).

In embodiments, the methods described herein are effective to reduce, delay, or prevent one or more other clinical symptoms of a depressive disorder, e.g., postpartum depression, seasonal affective disorder, persistent depressive disorder, premenstrual dysphoric disorder, psychotic depression, disruptive mood dysregulation disorder, refractory depression and/or depressive disorders due to another medical condition.

In embodiments, compositions and methods of treatment are provided with low dosages of gaboxadol or pharmaceutically acceptable salt thereof such that the patient is provided one or more beneficial effects related to a depressive disorder, such as, reduced sadness, reduced fatigue, increased mood, increased concentration, reduced insomnia, reduced irritability, reduced anxious or sad feelings, reduced feelings of worthlessness, reduced feelings of helplessness, reduced suicidal thoughts, increased behavioral control and/or increased cognitive ability. Provided herein are dosing regimens that allow effective treatment of a depressive disorder with potentially limited or substantially few negative side effects, *e.g.,* sleep disruption. For example, methods are provided herein of treating depressive disorders in a patient in need thereof which may not cause sleep disruption. In embodiments, methods described herein may provide effective treatment of a depressive disorder without interrupting Slow Wave Sleep. In embodiments methods of treating a depressive disorder without causing insomnia or trouble falling asleep are provided. In a proof of concept study, it was found that combining gaboxadol with escitalopram (Lexapro^{™}), a well-known antidepressant, provided no additional benefit over escitalopram alone in the treatment of patients with severe major depressive disorder. See, Kasper et al., Int J Neuropsychopharmacol. 2012 Jul;15(6):715-25. Accordingly, the methods described herein may provide treatment of a depressive disorder that may be considered surprising and unexpected.

The methods described herein may be useful for treating children, and for treating disorders that onset during infancy or childhood. In embodiments, the subject of the disclosed method is a toddler, a preschooler, a school-age child, a tween, or a teenager. In embodiments, the subject is 18 years old or younger, 12 years old or younger, 10 years old or younger, 8 years old or younger, 6 years old or younger, 4 years old or younger, 2 years old or younger, 1 year old or younger. In embodiments, the subject is an adult that is over eighteen years old.

In embodiments, gaboxadol is provided as gaboxadol monohydrate. One skilled in the art will readily understand that the amounts of active ingredient in a pharmaceutical composition will depend on the form of gaboxadol provided. For example, pharmaceutical compositions of including 5.0, 10.0, or 15.0 mg gaboxadol correspond to 5.6, 11.3, or 16.9 mg gaboxadol monohydrate.

In embodiments, gaboxadol is crystalline, such as the crystalline hydrochloric acid salt, the crystalline hydrobromic acid salt, or the crystalline zwitter ion monohydrate. In embodiments, gaboxadol is provided as a crystalline monohydrate.

In embodiments, methods of treating a depressive disorder include administering to a patient in need thereof a pharmaceutical composition including about 0.05 mg to about 50 mg gaboxadol or a pharmaceutically acceptable salt thereof. In embodiments, methods of treating a depressive disorder include administering to a patient in need thereof a pharmaceutical composition including about 0.1 mg to about 30 mg gaboxadol or a pharmaceutically acceptable salt thereof.

In embodiments, the pharmaceutical compositions include 0.1 mg to 25 mg, 0.1 mg to 20 mg, 0.1 mg to 15 mg, 0.5 mg to 25 mg, 0.5 mg to 20 mg, 0.5 to 15 mg, 1 mg to 25 mg, 1 mg to 20 mg, 1 mg to 15 mg, 1.5 mg to 25 mg, 1.5 mg to 20 mg, 1.5 mg to 15 mg, 2 mg to 25 mg, 2 mg to 20 mg, 2 mg to 15 mg, 2.5 mg to 25 mg, 2.5 mg to 20 mg, 2.5 mg to 15 mg, 3 mg to 25 mg, 3 mg to 20 mg, 3 mg to 15 mg gaboxadol or a pharmaceutically acceptable salt thereof.

In embodiments, the pharmaceutical compositions include 5 mg to 20 mg, 5 mg to 10 mg, 4 mg to 6 mg, 6 mg to 8 mg, 8 mg to 10 mg, 10 mg to 12 mg, 12 mg to 14 mg, 14 mg to 16 mg, 16 mg to 18 mg, or 18 mg to 20 mg gaboxadol or a pharmaceutically acceptable salt thereof.

In embodiments, the pharmaceutical compositions include 0.1 mg, 0.25 mg, 0.5 mg, 1 mg, 2.5 mg, 3 mg, 4 mg, 5 mg, 7 mg, 7.5 mg, 10 mg, 12.5 mg, 15 mg, 17.5 mg, 20 mg gaboxadol or a pharmaceutically acceptable salt thereof or amounts that are multiples of such doses. In embodiments, the pharmaceutical compositions include 2.5 mg, 5 mg, 7.5 mg, 10 mg, 15 mg, or 20 mg gaboxadol or a pharmaceutically acceptable salt thereof.

In embodiments, the total amount of gaboxadol or pharmaceutically acceptable salt thereof and/or gaboxadol administered to a subject in a 24-hour period is 1 mg to 50 mg. In embodiments, the total amount of gaboxadol or pharmaceutically acceptable salt thereof and/or gaboxadol administered to a subject in a 24-hour period is 1 mg to 20 mg. In embodiments, the total amount of gaboxadol or pharmaceutically acceptable salt thereof and/or gaboxadol administered to a subject in a 24-hour period is 5 mg, 10 mg, or 15 mg. In embodiments, the total amount of gaboxadol or a pharmaceutically acceptable salt thereof administered to a subject in a 24-hour period is 1 mg to 50 mg. In embodiments, the subject may be started at a low dose and the dosage is escalated. In this manner, it can be determined if the drug is well tolerated in the subject. Dosages can be lower for children than for adults. In embodiments, a dose of gaboxadol for children can be 0.1 mg/kg to 1 mg/kg.

In embodiments, provided herein are methods of treating a depressive disorder, e.g., MDD, postpartum depression, seasonal affective disorder, persistent depressive disorder, premenstrual dysphoric disorder, psychotic depression, disruptive mood dysregulation disorder, refractory depression, and/or depressive disorders due to another medical condition, which include administering to a patient in need thereof a pharmaceutical composition including gaboxadol or pharmaceutically acceptable salt thereof wherein the composition provides improvement in at least one symptom of the depressive disorder. In embodiments, the methods provided may also surprisingly and unexpectedly reduce or prevent symptoms of depression in a subject in need thereof. In embodiments, the methods provided may reduce or prevent one or more different types of depressive disorders.

In embodiments, methods described herein may reduce one or more symptoms of a depressive disorder in a subject after treatment compared to the absence of treatment (e.g., before treatment), or compared to treatment with an alternative conventional treatment.

In embodiments, provided herein are methods of treating a depressive disorder wherein the patient is provided improvement of at least one symptom for more than 4 hours after administration of the pharmaceutical composition to the patient. In embodiments, the improvement of at least one symptom for more than 6 hours after administration of the pharmaceutical composition to the patient is provided in accordance with the present disclosure. In embodiments, improvement of at least one symptom for more than, *e.g.,* 8 hours, 10 hours, 12 hours, 15 hours, 18 hours, 20 hours, or 24 hours after administration of the pharmaceutical composition to the patient is provided in accordance with the present disclosure. In embodiments, improvement in at least one symptom for at least *e.g.,* 8 hours, 10 hours, 12 hours, 15 hours, 18 hours, 20 hours, or 24 hours after administration of the pharmaceutical composition to the patient is provided in accordance with the present disclosure. In embodiments, improvement in at least one symptom for 12 hours after administration of the pharmaceutical composition to the patient is provided in accordance with the present disclosure.

In embodiments, provided herein are methods of treating a depressive disorder, e.g., MDD, postpartum depression, seasonal affective disorder, persistent depressive disorder, premenstrual dysphoric disorder, psychotic depression, disruptive mood dysregulation disorder, refractory depression, and/or depressive disorders due to another medical condition, which include administering gaboxadol or a pharmaceutical salt thereof to a patient in need thereof wherein the composition provides improvement in next day functioning to the patient.

In embodiments, provided herein methods of treating a depressive disorder, e.g., MDD, postpartum depression, seasonal affective disorder, persistent depressive disorder, premenstrual dysphoric disorder, psychotic depression, disruptive mood dysregulation disorder, refractory depression, and/or depressive disorders due to another medical condition, which include administering gaboxadol or a pharmaceutical salt thereof to a patient in need thereof wherein the patient is relieved of one or more symptoms of depression in about 2 weeks or less, 1 week or less, 1 day or less, or 1 hour or less (e.g. 15 minutes or less, half an hour or less), after administration. In embodiments, such methods may relieve the patient of at least one symptom of depression for about 1 day or more, 1 week or more, or 2 weeks or more after administration. In embodiments, provided herein is a method including parenterally administering an effective amount of gaboxadol or a pharmaceutical salt thereof to a patient suffering from depression, wherein the patient is substantially relieved of one or more symptoms of depression earlier after the first administration of gaboxadol or a pharmaceutical salt thereof, as compared to the same patient administered a different antidepressant compound.

In embodiments, provided herein are methods of treating a depressive disorder wherein the amount of active substance, e.g., gaboxadol or pharmaceutically acceptable salt thereof, within the patient about 4 hours after administration of the pharmaceutical composition is less than about 75% of the administered dose. In embodiments, provided herein are methods wherein the amount of gaboxadol or pharmaceutically acceptable salt thereof within the patient about, *e.g.,* 6 hours, 8 hours, 10 hours, 12 hours, 15 hours, or 20 hours after administration of the pharmaceutical composition is less than about 75%.

In embodiments, provided herein are methods of treating a depressive disorder wherein the amount of active substance, *e.g*., gaboxadol or pharmaceutically acceptable salt thereof within the patient about 4 hours after administration of the pharmaceutical composition is less than about 80% of the administered dose. In embodiments, provided herein are methods wherein the amount of active substance, *e.g*., gaboxadol or pharmaceutically acceptable salt thereof, within the patient about, *e.g.,* 6 hours, 8 hours, 10 hours, 12 hours, 15 hours, or 20 hours after administration of the pharmaceutical composition is less than about 80% of the administered dose.

In embodiments, provided herein are methods of treating a depressive disorder wherein the amount of active substance, *e.g*., gaboxadol or pharmaceutically acceptable salt thereof, within the patient about 4 hours after administration of the pharmaceutical composition is between about 65% to about 85% of the administered dose. In embodiments, the amount of active substance, *e.g*., gaboxadol or pharmaceutically acceptable salt thereof, within the patient after about, *e.g.,* 6 hours, 8 hours, 10 hours, 12 hours, 15 hours, or 20 hours after administration of the pharmaceutical composition is between about 65% to about 85% of the administered dose.

In embodiments, provided herein are methods of treating a depressive disorder including administering to a patient in need thereof a pharmaceutical composition including an active substance, *e.g*., gaboxadol or pharmaceutically acceptable salt thereof, wherein the composition provides an *in vivo* plasma profile having a Cₘₐₓ less than about 500 ng/ml. In embodiments, the composition provides improvement for more than 6 hours after administration to the patient.

In embodiments, the composition provides an *in vivo* plasma profile having a Cₘₐₓ less than about, *e.g.,* 450 ng/ml, 400 ng/ml 350 ng/ml, or 300 ng/ml and wherein the composition provides improvement of next day functioning of the patient. In embodiments, the composition provides an *in vivo* plasma profile having a Cₘₐₓ less than about, *e.g.,* 250 ng/ml, 200 ng/ml 150 ng/ml, or 100 ng/ml and wherein the composition provides improvement of next day functioning of the patient.

In embodiments, provided herein are methods of treating a depressive disorder including administering to a patient in need thereof a pharmaceutical composition wherein the composition provides a consistent *in vivo* plasma profile having a AUC_{0-∞} of less than about 900 ng•hr/ml. In embodiments, the composition provides improvement in next day functioning of the patient. In embodiments, the compositions provide an *in vivo* plasma profile having a AUC_{0-∞} of less than about, *e.g.,* 850 ng•hr/ml, 800 ng•hr/ml, 750 ng•hr/ml, or 700 ng•hr/ml and wherein the composition provides improvement of next day functioning of the patient. In embodiments, the composition provides improvement in one or more symptom for more than 6 hours after administration.

In embodiments, provided herein are methods of treating a depressive disorder including administering to a patient in need thereof a pharmaceutical composition comprising an active substance, *e.g*., gaboxadol or pharmaceutically acceptable salt thereof, wherein the composition provides an *in vivo* plasma profile having a AUC_{0-∞} of less than about, *e.g.,* 650 ng•hr/ml, 600 ng•hr/ml, 550 ng•hr/ml, 500 ng•hr/ml, or 450 ng•hr/ml. In embodiments, the composition provides an *in vivo* plasma profile having a AUC_{0-∞} of less than about, *e.g.,* 400 ng•hr/ml, 350 ng•hr/ml, 300 ng•hr/ml, 250 ng•hr/ml, or 200 ng•hr/ml. In embodiments, the composition provides an *in vivo* plasma profile having a AUC_{0-∞} of less than about, *e.g.,* 150 ng•hr/ml, 100 ng•hr/ml, 75 ng•hr/ml, or 50 ng•hr/ml. In embodiments, the composition provides improvement of next day functioning of the patient after administration for more than, *e.g.,* 4 hours, 6 hours, 8 hours, 10 hours, or 12 hours, after administration of the composition to the patient.

In embodiments, provided herein are methods of treating a depressive disorder including administering to a patient in need thereof a first pharmaceutical composition including gaboxadol or a pharmaceutically acceptable salt thereof and a second pharmaceutical composition including gaboxadol or a pharmaceutically acceptable salt thereof. In some embodiments, the second pharmaceutical composition provides an *in vivo* plasma profile having a mean AUC_{0-∞} of at least about 20% less than the first pharmaceutical composition.

In embodiments the first and/or the second pharmaceutical compositions are administered once, twice, or three times daily, or every other day. In embodiments, the first or the second pharmaceutical composition is provided to the patient in the evening. In embodiments, the second pharmaceutical composition includes an amount of gaboxadol that is at least one third of the amount of the gaboxadol or pharmaceutically acceptable salt thereof in the first pharmaceutical composition. In embodiments, the second pharmaceutical composition includes an amount of gaboxadol that is at least half of the amount of the amount of the gaboxadol or pharmaceutically acceptable salt thereof provided in the first pharmaceutical composition.

In embodiments, the first or the second pharmaceutical composition are provided to the patient once in the evening and once in the morning. In embodiments, the total amount of gaboxadol or pharmaceutically acceptable salt thereof administered to a subject in a 24-hour period is 1 mg to 100 mg. In embodiments, the total amount of gaboxadol or a pharmaceutically acceptable salt thereof administered to a subject in a 24-hour period is 1 mg to 75 mg. In embodiments, the total amount of active substance, *e.g*., gaboxadol or pharmaceutically acceptable salt thereof and/or gaboxadol, administered to a subject in a 24-hour period is less than about 75 mg, 50 mg, 25 mg, 20 mg, 10 mg, or 5 mg. In embodiments, the total amount of active substance, *e.g*., gaboxadol or pharmaceutically acceptable salt thereof and/or gaboxadol, administered to a subject in a 24-hour period is less than 15 mg.

In embodiments, gaboxadol or a pharmaceutically acceptable salt thereof may be administered in combination with an effective amount of one or more of a dopamine active anti-depressant agent, a dopamine active augmenting agent, a serotonin-a norepinephrine reuptake inhibitor (SNRT), a norepinephrine reuptake inhibitor, a monoamine oxidase inhibitor, a tricyclic antidepressant, a tetracyclic antidepressant, and a selective serotonin reuptake inhibitor (SSRI). Antidepressants include, but are not limited to, citalopram, escitalopram, paroxetine, fluvoxamine, sertraline, desvenlafaxine, duloxetine, levomilnacipran, milnacipran, tofenacin, venlafaxine, vilazodone, vortioxetine, etoperidone, trazodone, reboxetine, viloxazine, amitriptyline, amitriptylinoxide, clomipramine, desipramine, dibenzepin, dimetacrine, dosulepin, doxepin, imipramine, lofepramine, melitracen, nitroxazepine, nortriptyline, noxiptiline, pipofezine, protriptyline, trimipramine, amoxapine, maprotiline, mianserin, mirtazapine, setiptiline, socarboxazid, phenelzine, tranylcypromine, selegiline, metralindole, moclobemide, pirlindole, toloxatone, amisulpride, lurasidone, quetiapine, gomelatine, bifemelane, bupropion, ketamine, tandospirone and teniloxazine.

In embodiments, compositions herein are suitable for parenteral administration, including, *e.g*., intramuscularly (i.m.), intravenously (i.v.), subcutaneously (s.c.), intraperitoneally (i.p.), or intrathecally (i.t.). The parenteral compositions herein must be sterile for administration by injection, infusion or implantation into the body and may be packaged in either single-dose or multi-dose containers.

In embodiments, liquid pharmaceutical compositions for parenteral administration to a subject including gaboxadol or a pharmaceutically acceptable salt thereof at a concentration of about 0.005 µg/ml to about 500 µg/ml are provided. In embodiments, the composition includes gaboxadol or a pharmaceutically acceptable salt thereof at a concentration of, *e.g.,* about 0.005 µg/ml to about 250 µg/ml, about 0.005 µg/ml to about 200 µg/ml, about 0.005 µg/ml to about 150 µg/ml, about 0.005 µg/ml to about 100 µg/ml, or about 0.005 µg/ml to about 50 µg/ml.

In embodiments, compositions include gaboxadol or a pharmaceutically acceptable salt thereof at a concentration of, *e.g.,* about 0.05 µg/ml to about 50 µg/ml, about 0.1 µg/ml to about 50 µg/ml, about 0.05 µg/ml to about 25 µg/ml, about 0.05 µg/ml to about 10 µg/ml, about 0.05 µg/ml to about 5 µg/ml, or about 0.05 µg/ml to about 1 µg/ml. In embodiments, a composition includes gaboxadol or a pharmaceutically acceptable salt thereof at a concentration of, *e.g.,* about 0.05 µg/ml to about 15 µg/ml, about 0.5 µg/ml to about 10 µg/ml, about 0.5 µg/ml to about 7 µg/ml, about 1 µg/ml to about 10 µg/ml, about 5 µg/ml to about 10 µg/ml, or about 5 µg/ml to about 15 µg/ml. In embodiments, pharmaceutical compositions for parenteral administration are formulated as a total volume of about, *e.g.,* 10 ml, 20 ml, 25 ml, 50 ml, 100 ml, 200 ml, 250 ml, or 500 ml. In embodiments, compositions are contained in a bag, a glass vial, a plastic vial, or a bottle.

In embodiments, methods of treating a depressive disorder by administering to a patient in need thereof a parenteral pharmaceutical composition including gaboxadol or a pharmaceutically acceptable salt thereof at a concentration of about 0.05 µg/ml to about 500 µg/ml are provided. In embodiments, the composition is disposed within a sealed glass container.

In embodiments, compositions for parenteral administration including about 0.05 mg to about 100 mg gaboxadol or a pharmaceutically acceptable salt thereof are provided. In embodiments, the pharmaceutical compositions include about, *e.g.,* 0.1 mg to 25 mg, 0.1 mg to 20 mg, 0.1 mg to 15 mg, 0.5 mg to 25 mg, 0.5 mg to 20 mg, 0.5 to 15 mg, 1 mg to 25 mg, 1 mg to 20 mg, 1 mg to 15 mg, 1.5 mg to 25 mg, 1.5 mg to 20 mg, 1.5 mg to 15 mg, 2 mg to 25 mg, 2 mg to 20 mg, 2 mg to 15 mg, 2.5 mg to 25 mg, 2.5 mg to 20 mg, 2.5 mg to 15 mg, 3 mg to 25 mg, 3 mg to 20 mg, 3 mg to 15 mg gaboxadol or a pharmaceutically acceptable salt thereof.

In embodiments, the pharmaceutical compositions for parenteral administration include about, *e.g.,* 5 mg to 20 mg, 5 mg to 10 mg, 4 mg to 6 mg, 6 mg to 8 mg, 8 mg to 10 mg, 10 mg to 12 mg, 12 mg to 14 mg, 14 mg to 16 mg, 16 mg to 18 mg, or 18 mg to 20 mg gaboxadol or a pharmaceutically acceptable salt thereof. In embodiments, the pharmaceutical compositions for parenteral administration include about, *e.g.,* 0.1 mg, 0.25 mg, 0.5 mg, 1 mg, 2.5 mg, 3 mg, 4 mg, 5 mg, 7 mg, 7.5 mg, 10 mg, 12.5 mg, 15 mg, 17.5 mg, 20 mg gaboxadol or a pharmaceutically acceptable salt thereof or amounts that are multiples of such doses. The compositions may be contained in a bag, a glass vial, a plastic vial, or a bottle.

In embodiments, pharmaceutical compositions for parenteral administration to a subject include gaboxadol or a pharmaceutically acceptable salt thereof at a concentration of about 0.005 mg/ml to about 500 mg/ml. In embodiments, the compositions include gaboxadol or a pharmaceutically acceptable salt thereof at a concentration of, *e.g.,* about 0.05 mg/ml to about 50 mg/ml, about 0.05 mg/ml to about 100 mg/ml, about 0.005 mg/ml to about 500 mg/ml, about 0.1 mg/ml to about 50 mg/ml, about 0.1 mg/ml to about 10 mg/ml, about 0.05 mg/ml to about 25 mg/ml, about 0.05 mg/ml to about 10 mg/ml, about 0.05 mg/ml to about 5 mg/ml, or about 0.05 mg/ml to about 1 mg/ml. In embodiments, the composition includes gaboxadol or a pharmaceutically acceptable salt thereof at a concentration of, *e.g.,* about 0.05 mg/ml to about 15 mg/ml, about 0.5 mg/ml to about 10 mg/ml, about 0.25 mg/ml to about 5 mg/ml, about 0.5 mg/ml to about 7 mg/ml, about 1 mg/ml to about 10 mg/ml, about 5 mg/ml to about 10 mg/ml, or about 5 mg/ml to about 15 mg/ml. In embodiments, the pharmaceutical compositions for parenteral administration are formulated as a total volume of about, *e.g.,* 10 ml, 20 ml, 25 ml, 50 ml, 100 ml, 200 ml, 250 ml, or 500 ml. In embodiments, the compositions are packaged and stored in a bag, a glass vial, a plastic vial, or a bottle.

In embodiments, pharmaceutical compositions including gaboxadol or a pharmaceutically acceptable salt thereof wherein the gaboxadol or pharmaceutically acceptable salt thereof is present at a molarity less than about 1.0 M are provided. In embodiments, gaboxadol or pharmaceutically acceptable salt thereof is present at a molarity greater than, *e.g*., about 0.0001 M about 0.001 M, about 0.01 M, about 0.1 M, about 0.2 M, greater than about 0.5, greater than about 1.0 M, greater than about 1.2 M, greater than about 1.5 M, greater than about 1.75 M, greater than about 2.0 M, or greater than about 2.5 M. In embodiments, gaboxadol or pharmaceutically acceptable salt thereof is present at a molarity of between, *e.g*., about 0.00001 M to about 0.1 M, about 0.01 to about 0.1 M, about 0.1 M to about 1.0 M, about 1.0 M to about 5.0 M, or about 5.0 M to about 10.0 M. In embodiments, gaboxadol or pharmaceutically acceptable salt thereof is present at a molarity of less than, *e.g.,* about 0.01 M, about 0.1 M, about 1.0 M, about 5.0 M, or about 10.0 M

In embodiments, the solubility of gaboxadol or pharmaceutically acceptable salt thereof in the composition is greater than, *e.g*., about 10 mg/mL, about 15 mg/mL, about 20 mg/mL, about 25 mg/mL, about 30 mg/mL, about 40 mg/mL, about 50 mg/mL, about 75 mg/mL, about 100 mg/mL, about 150 mg/mL, when measured, for example, in water at 25°C.

In embodiments, the solubility of gaboxadol or pharmaceutically acceptable salt thereof in the composition is between, *e.g*., about 1 mg/mL to about 50 mg/mL, about 5 mg/mL to about 50 mg/mL, about 10 mg/mL to about 50 mg/mL, about 20 mg/mL to about 50 mg/ml, from about 20 mg/mL to about 30 mg/mL or from about 10 mg/mL to about 45 mg/mL, when measured, for example, in water at 25 C.

In embodiments, a pharmaceutical composition for parenteral administration is provided wherein the pharmaceutical composition is stable for at least six months. In embodiments, the pharmaceutical compositions herein exhibit no more than about 5% decrease in gaboxadol or pharmaceutically acceptable salt thereof after, *e.g.,* 3 months or 6 months. In embodiments, the amount of gaboxadol or pharmaceutically acceptable salt thereof degradation is no more than about, *e.g.,* 2.5%, 1%, 0.5% or 0.1%. In embodiments, the degradation of gaboxadol or pharmaceutically acceptable salt thereof is less than about, *e.g.,* 5%, 2.5%, 1%, 0.5%, 0.25%, 0.1%, for at least six months.

In embodiments, pharmaceutical compositions for parenteral administration wherein the pharmaceutical composition remains soluble are provided. In embodiments, pharmaceutical compositions that are stable, soluble, local site compatible and/or ready-to-use are provided. In embodiments, the pharmaceutical compositions herein are ready-to-use for direct administration to a patient in need thereof.

The parenteral compositions herein may include one or more excipients, *e.g*., solvents, solubility enhancers, suspending agents, buffering agents, isotonicity agents, stabilizers or antimicrobial preservatives. When used, the excipients of the parenteral compositions will not adversely affect the stability, bioavailability, safety, and/or efficacy of gaboxadol or pharmaceutically acceptable salt used in the composition. Thus, parenteral compositions are provided wherein there is no incompatibility between any of the components of the dosage form.

Thus, in embodiments, parenteral compositions of gaboxadol or a pharmaceutically acceptable salt thereof including a stabilizing amount of at least one excipient are provided. For example, excipients may be selected buffering agents, solubilizing agents, tonicity agents, antioxidants, chelating agents, antimicrobial agents, preservatives, and combinations thereof. One skilled in the art will appreciate that an excipient may have more than one function and be classified in one or more defined group.

In embodiments pharmaceutical compositions are provided including gaboxadol, or a pharmaceutically acceptable salt thereof, and an excipient wherein the excipient is present at a weight percent (w/v) of less than about, *e.g.,* 10%, 5%, 2.5%, 1%, or 0.5% are provided. In embodiments, the excipient is present at a weight percent between about, *e.g.,* 1.0% to 10%, 10% to 25%, 15% to 35%, 0.5% to 5%, 0.001% to 1%, 0.01% to 1%, 0.1% to 1%, or 0.5% to 1%. In embodiments, the excipient is present at a weight percent between about, *e.g.,* 0.001% to 1%, 0.01% to 1%, 1.0% to 5%, 10% to 15%, or 1% to 15%.

In embodiments pharmaceutical compositions are provided including gaboxadol, or a pharmaceutically acceptable salt thereof, and an excipient wherein the excipient is present in a molar ratio of the excipient to gaboxadol or pharmaceutically acceptable salt of, *e.g.,* about 0.01:1 to about 0.45: 1, about 0.1:1 to about 0.15:1, about 0.01:1 to about 0.1:1, and about 0.001:1 to about 0.01:1 are provided. In embodiments, the excipient is present at a molar ratio of the excipient to gaboxadol or pharmaceutically acceptable salt is about 0.0001:1 to about 0.1:1 or about 0.001:1 to about 0.001:1.

In embodiments, pharmaceutical compositions are provided including gaboxadol, or a pharmaceutically acceptable salt thereof and an excipient wherein the excipient comprises a stabilizing amount of a buffering agent. The buffering agent may be used to maintain the pH of the pharmaceutical composition wherein the gaboxadol or pharmaceutically acceptable salt thereof remains soluble, stable, and/or physiologically compatible. For example, in embodiments, the parenteral compositions include a buffering agent wherein the composition remains stable without significant gaboxadol degradation. In embodiments, the addition of a buffer is desired for controlling the pH to enhance stability without significantly catalyzing or degrading the gaboxadol or salt thereof and/or causing pain to the patient upon infusion.

In embodiments, the buffering agent can be a citrate, phosphate, acetate, tartrate, carbonate, glutamate, lactate, succinate, bicarbonate buffer and combinations thereof. For example, sodium citrate, trisodium citrate anhydrous, trisodium citrate dihydrate, sodium citrate dehydrate, triethanolamine (TRIS), trisodium citrate pentahydrate dihydrate (*i.e.,* trisodium citrate dehydrate), acetic acid, citric acid, glutamic acid, phosphoric acid, may be used as a buffering agent. In embodiments, the buffering agent may be an amino acid, alkali metal, or alkaline earth metal buffer. For example, the buffering agent may be sodium acetate or hydrogen phosphate.

In embodiments, provided herein are parenteral compositions of gaboxadol of pharmaceutically acceptable salts thereof wherein the pH of the composition is between about 4.0 to about 8.0. In embodiments, the pH of the compositions is between, *e.g*., about 5.0 to about 8.0, about 6.0 to about 8.0, about 6.5 to about 8.0. In embodiments, the pH of the compositions is between, *e.g*., about 6.5 to about 7.5, about 7.0 to about 7.8, about 7.2 to about 7.8, or about 7.3 to about 7.6. In embodiments, the pH of the aqueous solution of gaboxadol is, *e.g.,* about 6.8, about 7.0, about 7.2, about 7.4, about 7.6, about 7.7, about 7.8, about 8.0, about 8.2, about 8.4, or about 8.6.

In embodiments, pharmaceutical compositions are provided including gaboxadol, or a pharmaceutically acceptable salt thereof and an excipient wherein the excipient includes a solubilizing agent. For example, solubilizing agents according to the invention may include, *e.g*., sodium hydroxide, L-lysine, L-arginine, sodium carbonate, potassium carbonate, sodium phosphate, and/or potassium phosphate. The amount of solubilizing agent in the composition will be sufficient such that the solution remains soluble at all concentrations, *i.e.,* does not turn hazy and/or form precipitates.

In embodiments, provided herein are pharmaceutical compositions including gaboxadol, or a pharmaceutically acceptable salt thereof and an excipient wherein the excipient includes a particulate formation inhibitor. A particulate formation inhibitor refers to a compound that has the desired property of inhibiting the formation of particles in parenteral compositions. Particulate formation inhibitors of the invention include ethylenediaminetetraacetic acid (EDTA) and salts thereof, for example, ethylenediaminetetraacetic acid, calcium disodium salt (preferably as the hydrate); ethylenediaminetetraacetic acid, diammonium salt (preferably as the hydrate); ethylenediaminetetraacetic acid, dipotassium salt (preferably as the dihydrate); ethylenediaminetetraacetic acid, disodium salt (preferably as the dihydrate and, if desired, as the anhydrous form); ethylenediaminetetraacetic acid, tetrasodium salt (preferably as the hydrate); ethylenediaminetetraacetic acid, tripotassium salt (preferably as the dihydrate); ethylenediaminetetraacetic acid, trisodium salt (preferably as the hydrate) and ethylenediaminetetraacetic acid disodium salt, USP(preferably as the dihydrate). In embodiments, pharmaceutical compositions described herein have an effective amount of a particulate formation inhibitor. In embodiments the excipients may include, e.g., an amino acid, urea, alcohol, ascorbic acid, phospholipids, proteins, such as serum albumin, collagen, and gelatin; salts such as EDTA or EGTA, and sodium chloride, liposomes, polyvinylpyrollidone, sugars, such as dextran, mannitol, sorbitol, and glycerol, propylene glycol and polyethylene glycol (*e.g.,* PEG-4000, PEG-6000), glycerol, glycine, and/or lipids.

In embodiments, provided herein are pharmaceutical compositions including gaboxadol, or a pharmaceutically acceptable salt thereof and an excipient wherein the excipient includes a solubilizing agent. For example, solubilizing agents may include, but are not limited to, acids, such as carboxylic acids, amino acids. In other examples, the solubilizing agents may be saturated carboxylic acids, unsaturated carboxylic acids, fatty acids, keto acids, aromatic carboxylic acids, dicarboxylic acids, tricarboxylic acids, α-hydroxy acids, amino acids, and combinations thereof.

In embodiments, provided herein are pharmaceutical compositions including gaboxadol or a pharmaceutically acceptable salt thereof and an excipient wherein the excipient includes a solubilizing agent such as formic acid, acetic acid, propionic acid, butyric acid, valeric acid, caproic acid, enanthic acid, caprylic acid, pelargonic acid, capric acid, lauric acid, stearic acid, acrylic acid, docosahexaenoic acid, eicosapentaenoic acid, pyruvic acid, benzoic acid, salicylic acid, aldaric acid, oxalic acid, malonic acid, malic acid, succinic acid, glutaric acid, adipic acid, citric acid, lactic acid, alanine, arginine, aspargine, aspartic acid, cysteine, glutamine, glycine, histidine, isoleucine, leucine, lysine, methionine, phenylalanine, praline, serine, threonine, tryptophan, tyrosine, valine, and combinations thereof.

In embodiments, the solubilizing agent is selected from acetic acid, salts thereof, and combinations thereof, (*e.g.,* acetic acid/sodium acetate), citric acid, salts thereof and combinations thereof (*e.g*., citric acid/sodium citrate), DL arginine, L-arginine and histadine. In embodiments, the solubilizing agent is DL-arginine. In embodiments, the solubilizing agent is L-arginine. In embodiments, the solubilizing agent is acetic acid/sodium acetate. In embodiments, the solubilizing agent is citric acid/sodium citrate.

In embodiments, provided herein are pharmaceutical compositions including gaboxadol or a pharmaceutically acceptable salt thereof and an excipient wherein the excipient renders the composition isotonic. Isotonic pharmaceutical compositions herein may be achieved by adding an appropriate quantity of sodium chloride, glucose, laevulose, dextrose, mannitol, or postassium chloride, or calcium chloride, or calcium gluconoglucoheptonate, or mixtures thereof. For example, the excipients may include one or more tonicity agents, such as, *e.g*., sodium chloride, potassium chloride, glycerin, mannitol, and/or dextrose. Tonicity agents may be used to minimize tissue damage and irritation, reduce hemolysis of blood cells, and/or prevent electrolyte imbalance. For example, the parenteral compositions may be an aqueous solution comprising sodium chloride wherein the composition is isotonic. In embodiments, the isotonizing agent is sodium chloride. In embodiments, the concentration of the isotonizing agent is between about 0.01 and about 2.0 weight percent. In embodiments, the pharmaceutical compositions may comprise up to about 10% isotonizing agent. In embodiments the pharmaceutical compositions may comprise up to about, *e.g.,* 0.25%, 0.5%, 1%, 2.5% isotonizing agent. In embodiments the amount of isotonizing agent in the pharmaceutical is between about, *e.g.,* 0.01% to 1%, 0.1% to 1%, 0.25% to 1%, or 0.5% to 1%.

In embodiments, provided herein are pharmaceutical compositions including gaboxadol, or a pharmaceutically acceptable salt thereof and an excipient wherein the excipient includes a free radical antagonist. In embodiments, the free radical antagonist is ascorbic acid, ascorbic acid derivatives, organic compounds having at least one thiol, alkyl polyhydroxylated, and cycloalkyl polyhydroxylated compounds, and combinations thereof.

In embodiments, provided herein are pharmaceutical compositions including gaboxadol, or a pharmaceutically acceptable salt thereof and an excipient wherein the excipient includes a free radical scavenger selected from thiolyglycolic acid, thiolacetic acid, dithiothreitol, reduced glutathion, thiourea, α-thioglycerol, cystein, acetlcystein, mercaptoethane sulfonic acid and combinations thereof.

In embodiments, provided herein are pharmaceutical compositions including gaboxadol, or a pharmaceutically acceptable salt thereof and an excipient wherein the excipient includes ribolflavin, dithiothreitol, sodium thiosulfate, thiourea, ascorbic acid, methylene blue, sodium metabisulfite, sodium bisulfite, propyl gallate acetylcysteine, phenol, acetone sodium bisulfate, ascorbic acid, ascorbic acid esters, butylhydroxyanisol (BHA), Butylhydroxytoluene (BHT), cysteine, nordihydroguiaretic acid (NDGA), monothioglycerol, sodium bisulfite, sodium metabisulfate, tocophenols, and/or glutathione.

In embodiments, provided herein are pharmaceutical compositions including gaboxadol, or a pharmaceutically acceptable salt thereof and an excipient wherein the excipient includes a preservative. In embodiments, the preservative is selected from benzalkonium chloride, benzethonium chloride, benzyl alcohol, chlorobutanol, chlorocresol, metacresol, Phenol, phenylmercuric nitrate, phenylmercuric acetate, methyl p-hydroxybenzoate, propyl p-hydroxybenzoate, butyl p-hydroxybenzoate, and thimerosal. In other embodiments, the preservative is selected from the group consisting of phenol, metacresol, benzyl alcohol, parabens (e.g., methyl, propyl, butyl), benzalkonium chloride, chlorobutanol, thimerosal, phenylmercuric salts (e.g., acetate, borate, or nitrate), and combinations thereof.

In embodiments, the compositions herein include a co-solvent. In some instances the solubility of gaboxadol may be well below the therapeutic dose and therefore a co-solvent system may be used. A co-solvent is a mixture of solvents that may be used to achieve sufficiently high solubility and may increase the stability. For example, co-solvents may be a water-miscible organic solvents, such as ethanol, propylene, glycol, Capmul PG, propylene glycol, glycerin, polyethylene glycol, sorbitol, dimethylacetamide, and/or dimethylsulfoxide (DMSO). In embodiments, the cosolvent may comprise up to about 75% of the pharmaceutical composition. In other embodiments the amount of cosolvent used include up to about, *e.g.,* 1%, 5%, 10%, 15%, 25%, 40%, 50%, of the pharmaceutical composition.

The dosage forms may be prepared, for example, by mixing gaboxadol and one or more excipients (e.g., buffering agents, solubilizing agents, tonicity agents, antioxidants, chelating agents, antimicrobial agents and/or preservatives) in a blender under sterile conditions until a uniform blend is obtained. Pre-sterilized vials may then be filled with an appropriate amount of the sterile blend. The predetermined amount of sterile blend may then be mixed with a solvent, *e.g.,* water, saline, about 5-10% sugar (*e.g.,* glucose, dextrose) solution and combinations thereof prior to administration. In addition, the solution may be frozen and thawed prior to further processing.

The excipients may be used in solid or in solution form. When used in solid form, the excipients and gaboxadol may be mixed together as described above, and then solvent added prior to parenteral administration. When used in solution form, the gaboxadol may be mixed with a solution of the excipient prior to parenteral administration.

Parenteral solutions comprising gaboxadol herein, may be prepared by mixing the required amount of gaboxadol which may be purified prior to use in parenteral fluids such as D5W, distilled water, saline or PEG and adjusting the pH of this solution between 6.8-8. The process may be carried out at room temperature, or to increase concentration, the solution may be warmed appropriately. Other solvents such as PEG 400, 600, polypropylene glycol or other glycols can be used to enhance solubility. The resulting solutions after cooling to room temperature, may be sterilized by known means such as ultrafiltration using, *e.g.,* 0.45 micron filter or ethylene oxide treatment or heating and may be packaged into ampules, vials or prefilled syringes suitable for dispensing a sterile parenteral formulation.

When administered, the parenteral compositions herein provide a time of maximum plasma concentration (Tₘₐₓ) for gaboxadol in human patients of about 1 or more hours (*e.g.,* about 1.5 or more hours). In embodiments, a Tₘₐₓ of gaboxadol in human patients ranging from between, *e.g*., about 1 to about 5 hours, about 1 to about 4 hours, about 1 to about 3 hours, about 1 to about 2 hours. In embodiments, a Tₘₐₓ for gaboxadol in human patients of more than about 1.5 is observed. In embodiments, a Tₘₐₓ for gaboxadol in human patients of less than about 3 hours is observed. The time of maximum plasma concentration is measured once infusion is complete.

In embodiments herein a dosage form includes from about 1 mg to about 500 mg gaboxadol, wherein parenteral administration (*e.g*., intramuscular, intravenous, subcutaneous, intraperitoneal, or intrathecal) of the dosage form provides an *in vivo* plasma profile for gaboxadol comprising a mean AUC_{0-∞} of more than about 25 ng•hr/ml. In embodiments, single dose administration of the dosage form provides an *in vivo* plasma profile for gaboxadol comprising a mean AUC_{0-∞} of more than about, *e.g.,* 50 ng•hr/ml, 75 ng•hr/ml, 150 ng•hr/ml, 250 ng•hr/ml, 500 ng•hr/ml, 1000 ng•hr/ml, or 1500 ng•hr/ml.

In embodiments, the dosage form includes from about 1 mg to about 500 mg gaboxadol, wherein administration of the dosage form provides an *in vivo* plasma profile for gaboxadol comprising a mean Cₘₐₓ of less than about 10000 ng/ml. In embodiments, single dose administration of the compositions provide an *in vivo* plasma profile for gaboxadol of a mean Cₘₐₓ of less than about, *e.g.,* 5000 ng/ml, 2500 ng/ml, 1000 ng/ml, 500 ng/ml, 250 ng/ml, or 100 ng/ml.

In embodiments, pharmaceutical compositions for parenteral administration include gaboxadol or a pharmaceutically acceptable salt thereof wherein parenteral administration exhibits a pharmacokinetic profile of a Tₘₐₓ at about 1 to about 120 minutes after administration of the parenteral composition; followed by a plasma drug concentration of at least 50% Cₘₐₓ for a duration of about 90 to about 360 minutes. In embodiments, parenteral administration of gaboxadol is followed by a plasma drug concentration of at least 50% Cₘₐₓ for a duration of, *e.g.,* about 10 to about 60 minutes, about 15 to about 90 minutes, about 30 to about 120 minutes, about 60 to about 180 minutes, about 90 to about 180 minutes.

In embodiments, stable pharmaceutical compositions are provided in unit dosage form in a vial or ampoule suitable for parenteral administration having a therapeutically effective amount of gaboxadol or pharmaceutically acceptable salt thereof dissolved in sterile water to form a solution wherein the composition is substantially free of any excipient, organic solvent, buffer, acid, base, salt other than gaboxadol or pharmaceutically acceptable salt thereof. In embodiments, the pharmaceutical composition remains sufficiently soluble and is capable of direct administration. In embodiments, the pharmaceutical composition is capable of storage in the absence of an inert atmosphere for at least 6 months.

In embodiments, provided herein are stable pharmaceutical compositions in unit dosage form in a vial or ampoule suitable for parenteral administration having a therapeutically effective amount of gaboxadol or pharmaceutically acceptable salt thereof dissolved in sterile water to form a solution wherein the composition is free of any excipient, organic solvent, buffer, acid, base, salt other than gaboxadol or pharmaceutically acceptable salt thereof. In embodiments, the pharmaceutical composition remains sufficiently soluble and is capable of direct administration. In embodiments, the pharmaceutical composition is capable of storage in the absence of an inert atmosphere for at least 6 months.

In embodiments, stable pharmaceutical compositions suitable for parenteral administration include gaboxadol or a pharmaceutically acceptable salt thereof, in an aqueous solution having an osmolarity between 225 and 350 mOsm/kg and at a pH in the range between 7.0 and 8.0. In embodiments, the aqueous solution has an osmolarity between 270 and 310. In embodiments, the aqueous solution has a pH in the range between 7.2 and 7.8.

Unless defined otherwise, all technical and scientific terms used herein have the same meanings as commonly understood by one of skill in the art to which the disclosed invention belongs.

Gaboxadol may be formulated for administration to a patient using pharmaceutically acceptable salts including acid addition salts, a zwitter ion hydrate, zwitter ion anhydrate, hydrochloride or hydrobromide salt, or in the form of the zwitter ion monohydrate. Acid addition salts, include but are not limited to, maleic, fumaric, benzoic, ascorbic, succinic, oxalic, bis-methylenesalicylic, methanesulfonic, ethane-disulfonic, acetic, propionic, tartaric, salicylic, citric, gluconic, lactic, malic, mandelic, cinnamic, citraconic, aspartic, stearic, palmitic, itaconic, glycolic, p-amino-benzoic, glutamic, benzene sulfonic or theophylline acetic acid addition salts, as well as the 8-halotheophyllines, for example 8-bromotheophylline. In other suitable embodiments, inorganic acid addition salts, including but not limited to, hydrochloric, hydrobromic, sulfuric, sulfamic, phosphoric or nitric acid addition salts may be used.

"Excipient" is a substance, other than the active drug substance, *e.g*., gaboxadol, of a pharmaceutical composition, which has been appropriately evaluated for safety and are included in a drug delivery system to either aid the processing of the drug delivery system during its manufacture; protect; support; enhance stability, bioavailability, or patient acceptability; assist in product identification; or enhance any other attributes of the overall safety and effectiveness of the drug delivery system during storage or use.

"Stabilizer" or "stabilizing amount" refers to an amount of one or more excipients included in the parenteral compositions that provide sufficient stability but do not adversely affect the bioavailability, safety and/or efficacy of gaboxadol or pharmaceutically acceptable salt used in the composition.

"Stable" means that there is substantially no degradation of the gaboxadol or pharmaceutically acceptable salt thereof after a specified period of time, *e.g.,* after 3 months or 6 months.

"Soluble" means that the solution of gaboxadol does not turn hazy and/or there is substantially no precipitate in the solution

"Sufficiently soluble" means that the particle content is sufficiently low, and the material is sufficiently sterile such that it is useful for parenteral administration. For example, the number of particles in a liquid composition should be, *e.g.,* less than 6,000 10 µm particles should be present in a volume of 10 ml solvent, preferably less than 10,000, less than 5,000, less than 3,000, less than 1,000, or less than 400 10 µm particles. In some examples, the number of particles in a liquid composition should be less than 1000, less than 600, or less than 200 25 µm particles in the 10 ml volume.

"Local site compatible" herein shall mean the composition is tolerant at the site of injection or infusion, thus minimizing side effects, such as local skin irritations or venous irritations, including inflammatory reactions at the infusion site. The parenteral compositions herein may have less side reactions than conventional products, such as skin irritation or phlebitis.

"Treating" or "treatment" refers to alleviating or delaying the appearance of clinical symptoms of a disease or condition in a subject that may be afflicted with or predisposed to the disease or condition, but does not yet experience or display clinical or subclinical symptoms of the disease or condition. In certain embodiments, "treating" or "treatment" may refer to preventing the appearance of clinical symptoms of a disease or condition in a subject that may be afflicted with or predisposed to the disease or condition, but does not yet experience or display clinical or subclinical symptoms of the disease or condition. "Treating" or "treatment" also refers to inhibiting the disease or condition, *e.g*., arresting or reducing its development or at least one clinical or subclinical symptom thereof. "Treating" or "treatment" further refers to relieving the disease or condition, *e.g*., causing regression of the disease or condition or at least one of its clinical or subclinical symptoms. The benefit to a subject to be treated may be statistically significant, mathematically significant, or at least perceptible to the subject and/or the physician. Nonetheless, prophylactic (preventive) and therapeutic (curative) treatment are two separate embodiments of the invention.

"Pharmaceutically acceptable" refers to molecular entities and compositions that are "generally regarded as safe, *e.g.,* that are physiologically tolerable and do not typically produce an allergic or similar untoward reaction, such as gastric upset and the like, when administered to a human. In embodiments, this term refers to molecular entities and compositions approved by a regulatory agency of the federal or a state government, as the GRAS list under section 204(s) and 409 of the Federal Food, Drug and Cosmetic Act, that is subject to premarket review and approval by the FDA or similar lists, the U.S. Pharmacopeia or another generally recognized pharmacopeia for use in animals, and more particularly in humans.

"Purified" as used herein refers to material that has been isolated under conditions that reduce or eliminate the presence of unrelated materials, *i.e.,* contaminants, including native materials from which the material is obtained. As used herein, the term "substantially free" is used operationally, in the context of analytical testing of the material. Preferably, purified material substantially free of contaminants is at least 95% pure; more preferably, at least 97% pure, and more preferably still at least 99% pure. Purity can be evaluated, for example, by chromatography or any other methods known in the art. In embodiments, purified means that the level of contaminants is below a level acceptable to regulatory authorities for safe administration to a human or non-human animal.

"Ready-to-use" with reference to the compositions herein shall mean the preparation in the reconstituted form, with standardized concentration and quality, prefilled in the singleuse container, such as glass vials, infusion bags or syringes, ready for direct administration to the patient.

"Direct administration" with reference to the compositions herein shall mean the immediate administration, *i.e.,* without further dilution, premixing with other substances or otherwise changing the composition or formulation of the composition. Such composition is typically directly discharged from an infusion device and administered via a vascular access port or through a central line.

"Dosage" is intended to encompass a formulation expressed in terms of µg/kg/day, µg/kg/hr, mg/kg/day or mg/kg/hr. The dosage is the amount of an ingredient administered in accordance with a particular dosage regimen. A "dose" is an amount of an agent administered to a mammal in a unit volume or mass, *e.g*., an absolute unit dose expressed in mg or µg of the agent. The dose depends on the concentration of the agent in the formulation, *e.g*., in moles per liter (M), mass per volume (m/v), or mass per mass (m/m). The two terms are closely related, as a particular dosage results from the regimen of administration of a dose or doses of the formulation. The particular meaning in any case will be apparent from context.

"About" or "approximately" as used herein means within an acceptable error range for the particular value as determined by one of ordinary skill in the art, which will depend in part on how the value is measured or determined, i.e., the limitations of the measurement system. For example, "about" can mean within 3 or more than 3 standard deviations, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value.

"Patient" and "subject" are used interchangeably herein and include, but not limited to, primates, canines, porcine, ungulates, rodents, poultry, and avian.

The Examples herein are included for purposes of illustration and should not be construed as limiting the disclosure herein.

### EXAMPLES

### Example 1

### Solubility Evaluation of Gaboxadol

Gaboxadol may exist as either an anhydrous zwitterion or as a monohydrate. The solid phase that can exist in equilibrium with a solution will necessarily depend on the water activity in the solution. If an excess amount of gaboxadol is added to water, the excess is precipitated as solid gaboxadol monohydrate, but if an excess amount of gaboxadol is added to organic solvents with low water content such as methanol, ethanol and isopropanol, the solid precipitate will be anhydrous gaboxadol. The solubility of gaboxadol versus pH has been determined and the calculated curves and measured values are shown in Figure 1. Since the lowest aqueous solubility measured is greater than 10 mg/ml, solubility is not considered a limiting factor for absorption.

As the solubility is defined as the concentration in a solution in equilibrium with the solid, the solubility determined in organic solvents will be the solubility of the anhydrate and not of the monohydrate. Therefore, the solubility of gaboxadol monohydrate was determined in water/organic solvent mixtures. The concentration of the drug substance as gaboxadol monohydrate was determined by liquid chromatography. The solubility of gaboxadol monohydrate in water and water-organic solvent mixtures measured in mg per ml is provided in Table 1.

**Table 1. Solubility of Gaboxadol Monohydrate in Water and Water-Organic Solvent Mixtures**

| **Solvent** | **Solubility (mg/ml)** |
|---|---|
| Water | 21.4 |
| 1:1 Water /Methanol (v/v) | 4.6 |
| 1:1 Water/Ethanol (absolute) (v/v) | 3.2 |
| 1:1 Water/Acetonitrile (v/v) | 4.2 |
| 1:1 Water/2-Propanol (v/v) | 2.2 |

The solubility in water versus pH measured in mg of gaboxadol monohydrate per ml are provided in Table 2.

**Table 2. Solubility of Gaboxadol Monohydrate in Water**

| **pH** | **Solubility (mg/ml)** |
|---|---|
| 4.7 | 33.7 |
| 5.2 | 23.5 |
| 5.5 | 21.8 |
| 6.4 | 21.4 |
| 6.8 | 22.0 |
| 7.2 | 23.9 |
| 7.5 | 26.5 |
| 7.8 | 30.1 |

### Example 2

### Assessment of Antidepressant Efficacy of Gaboxadol

This prospective study will be used to evaluate the dose-dependent ability of gaboxadol to relieve symptoms of depression in adults. Specifically, gaboxadol will be compared to placebo and will assess the magnitude and rate of response to parenteral gaboxadol, as measured by change on the Hamilton Rating Scale for Depression (HAM-D-17 or HAM-D-28), compared to placebo. Additional assessments will be based on the Montgomery-Asberg Depression Rating Scale (MADRS-10 or MADRS-15) and Clinical Global Impressions--Severity and Improvement (CGI-S and CGI-I).

The trial will be conducted over 6 weeks, with double-blind treatment based on random assignment to gaboxadol or placebo. To assess longevity of response to gaboxadol, patients assigned to active drug will be followed for 6 weeks, the placebo group will be followed for 3 weeks. Inclusion criteria will include an age range of 18-65; written informed consent; patients must meet DSM-IV criteria (by Structured Clinical Interview for DSM-IV SCID-UP) for MDD, current; Quick Inventory of Depressive Symptomatology--Self-Rated (QIDS-SR) score of at least 12 at both screen and baseline visits; will have been treated with SSRI in combination with a dopaminergic agent; or on an antidepressant with a dopaminergic mechanism of action, including SNRIs, MAOIs, TCAs, or bupropion, in adequate doses, will have achieved remission per ACNP Task Force guidelines (REF) for ≥3 months, currently in relapse or recurrence without dose change for at least the past 4 weeks, based on meeting DSM-IV criteria for MDD. During the baseline visit, patients will have been on a stable dose of antidepressant regimen for the previous 4 weeks.

Once patients agree to participate in the study by signing an informed consent document, a full medical and psychiatric history will be taken and a physical examination will be performed by a board-certified psychiatrist. Screen rating scales will be performed. Screened and eligible patients will be asked to return one week later for a baseline visit when they will be randomized to double-blind treatment with placebo or gaboxadol, with the study design outlined above. The patients will be assessed weekly. Subjects will be assigned randomization numbers in consecutive order. The randomization list will be provided by a computer-generated random-number list and will be maintained by the study clinicians. In addition, the presence of any side effect or adverse event will be carefully documented with the SAFTEE-SI. Reasons for premature discontinuation, including change in primary medications, will be recorded. All concomitant medications taken during the study will be recorded in the case report form, along with dosage information and start and stop dates. Medication management and clinical ratings will be performed by the study clinicians.

At the end of the double-blind study, both responders and non-responders who will have completed the double-blind phase will have the option of receiving open-label adjunctive treatment with gaboxadol. Subjects who agree to receive open-label treatment with gaboxadol for 3 months will be seen monthly by a board-certified psychiatrist until the end of the follow-up phase.

The primary efficacy measurement will be the change in 17-item Hamilton Rating Scale for Depression (HAM-D-17) score or the 28-item Hamilton Rating Scale for Depression (HAM-D-28). Secondary measures of efficacy will include change in CGIseverity, with clinical or CGI-improvement (CGI-I). An additional measure of efficacy will be the change in Montgomery-Asberg Depression Rating Scale (MADRS-10 or MADRS-15).

The following instruments will be administered according to the study schedule: a structured clinical interview for DSM-IV, an antidepressant treatment history questionnaire, the 17-item or 28-item Hamilton Depression Scale (HAM-D-17 or HAM-D-28), Clinical Global Impressions--severity and improvement (CGI-S, CGI-I), 10-item or 15-item Montgomery-Asberg Depression Rating Scale (MADRS-10 or MADRS-15), Quick inventory Depressive Symptomatology (Self Report) (QIDS-SR), a cognitive and physical functioning questionnaire, a sexual functioning questionnaire, the Quality of Life Satisfaction Questionnaire-short form (Q-LES-Q), and the Sheehan Disability Scale.

Patients will be examined to show specific efficacy for the treatment of "breakthrough depression," ("BTD") an umbrella term, which encompasses depressive relapse (a depressive episode within 6 months of antidepressant response) and recurrence (a depressive episode after 6 months of response). Additionally, patients with BTD on an antidepressant regimen containing a pro-dopaminergic agent assigned to treatment with gaboxadol will be assessed for significant differences in the number of adverse events, as measured by the SAFTEE-SI as well as greater improvement in Quality of Life, Enjoyment, and Satisfaction Questionnaire (Q-LES-Q) and Sheehan Disability Scale (SDS) scores, compared to placebo controls.

### Example 3

### Assessment of Antidepressant Efficacy of Gaboxadol on Patients with FMR1 Premutation Syndrome

This prospective study will be used to evaluate the dose-dependent ability of gaboxadol to relieve symptoms of depression in adults with *FMR1* premutation syndrome. Specifically, gaboxadol will be compared to placebo and will assess the magnitude and rate of response to parenteral gaboxadol, as measured by change on the Hamilton Rating Scale for Depression (HAM-D-17 or HAM-D-28), compared to placebo. Additional assessments will be based on the Montgomery-Asberg Depression Rating Scale (MADRS-10 or MADRS-15) and Clinical Global Impressions--Severity and Improvement (CGI-S and CGI-I).

The trial will be conducted over 6 weeks, with double-blind treatment based on random assignment to gaboxadol or placebo. To assess longevity of response to gaboxadol, patients assigned to active drug will be followed for 6 weeks, the placebo group will be followed for 3 weeks. Inclusion criteria will include an age range of 18-65; a *FMR1* CGG segment repeated between 55 to 200 times (*FMR1* CGG repeat lengths will be quantified in all subjects using conventional procedures), written informed consent; patients must meet DSM-IV criteria (by Structured Clinical Interview for DSM-IV SCID-UP) for MDD, current; Quick Inventory of Depressive Symptomatology--Self-Rated (QIDS-SR) score of at least 12 at both screen and baseline visits; will have been treated with SSRI in combination with a dopaminergic agent; or on an antidepressant with a dopaminergic mechanism of action, including SNRIs, MAOIs, TCAs, or bupropion, in adequate doses, will have achieved remission per ACNP Task Force guidelines (REF) for ≥3 months, currently in relapse or recurrence without dose change for at least the past 4 weeks, based on meeting DSM-IV criteria for MDD. During the baseline visit, patients will have been on a stable dose of antidepressant regimen for the previous 4 weeks.

Once patients agree to participate in the study by signing an informed consent document, a full medical and psychiatric history will be taken and a physical examination will be performed by a board-certified psychiatrist. Screen rating scales will be performed. Screened and eligible patients will be asked to return one week later for a baseline visit when they will be randomized to double-blind treatment with placebo or gaboxadol, with the study design outlined above. The patients will be assessed weekly. Subjects will be assigned randomization numbers in consecutive order. The randomization list will be provided by a computer-generated random-number list and will be maintained by the study clinicians. In addition, the presence of any side effect or adverse event will be carefully documented with the SAFTEE-SI. Reasons for premature discontinuation, including change in primary medications, will be recorded. All concomitant medications taken during the study will be recorded in the case report form, along with dosage information and start and stop dates. Medication management and clinical ratings will be performed by the study clinicians.

At the end of the double-blind study, both responders and non-responders who will have completed the double-blind phase will have the option of receiving open-label adjunctive treatment with gaboxadol. Subjects who agree to receive open-label treatment with gaboxadol for 3 months will be seen monthly by a board-certified psychiatrist until the end of the follow-up phase.

The primary efficacy measurement will be the change in 17-item Hamilton Rating Scale for Depression (HAM-D-17) score or the 28-item Hamilton Rating Scale for Depression (HAM-D-28). Secondary measures of efficacy will include change in CGIseverity, with clinical or CGI-improvement (CGI-I). An additional measure of efficacy will be the change in Montgomery-Asberg Depression Rating Scale (MADRS-10 or MADRS-15).

The following instruments will be administered according to the study schedule: a structured clinical interview for DSM-IV, an antidepressant treatment history questionnaire, the 17-item or 28-item Hamilton Depression Scale (HAM-D-17 or HAM-D-28), Clinical Global Impressions--severity and improvement (CGI-S, CGI-I), 10-item or 15-item Montgomery-Asberg Depression Rating Scale (MADRS-10 or MADRS-15), Quick inventory Depressive Symptomatology (Self Report) (QIDS-SR), a cognitive and physical functioning questionnaire, a sexual functioning questionnaire, the Quality of Life Satisfaction Questionnaire-short form (Q-LES-Q), and the Sheehan Disability Scale.

Those skilled in the art will recognize, or be able to ascertain using no more than routine experimentation, many equivalents to the specific embodiments described herein. Such equivalents are intended to be encompassed by the claims.

## Claims

1. A composition comprising gaboxadol or a pharmaceutically acceptable salt thereof for use in a method of treating FMR1-premutation syndrome, the method comprising administering the composition to a patient in need thereof such as to administer to the patient 1 mg to 30 mg gaboxadol or pharmaceutically acceptable salt.

2. The composition for use according to claim 1, wherein the patient is administered 10 mg gaboxadol or pharmaceutically acceptable salt per day.

3. The composition for use according to claim 1, wherein the patient is administered 15 mg gaboxadol or pharmaceutically acceptable salt per day.

4. The composition for use according to claim 1, wherein the patient is administered 20 mg gaboxadol or pharmaceutically acceptable salt per day.

5. The composition for use according to claim 1, wherein the composition is administered once daily.

6. The composition for use according to claim 1, wherein the composition is administered twice daily.

7. The composition for use according to claim 1, wherein the dose of gaboxadol or pharmaceutically acceptable salt administered to the patient is 0.1-20 mg/day.

8. The composition for use according to claim 1, wherein the dose of gaboxadol or pharmaceutically acceptable salt administered to the patient is 0.2-15 mg/day.

9. The composition for use according to claim 1, wherein the dose of gaboxadol or pharmaceutically acceptable salt administered to the patient is 0.5-10 mg/day.

10. The composition for use according to claim 1, wherein the composition is administered parenterally, preferably intravenously.

11. The composition for use according to claim 1, wherein the composition is a liquid composition.
